# EUROPEAN PATENT APPLICATION

(11) **EP 4 737 471 A1**
(43) Date of publication of application: **06.05.2026**
(21) Application number: 24209560.2
(22) Date of filing: 29.10.2024
(51) Int. Cl.: C07K 1/00, C07K 14/00, C07K 1/107

(54) **METHODS FOR THE C-TERMINAL MODIFICATION OF (POLY)PEPTIDES**

(71) Applicant: NUMAFERM GmbH, 40225 Düsseldorf (DE)
(72) Inventor: Schwarz, Christian, 40225 Düsseldorf (DE); Rebmann, Philipp, 40225 Düsseldorf (DE); Strauß, Jannik, 40225 Düsseldorf (DE); Seide, Selina, 40225 Düsseldorf (DE)
(74) Representative: Maiwald GmbH

(57) **Abstract**

The present invention concerns a method for the C-terminal modification of a (poly)peptide that is free of cysteine residues comprising providing the to-be-modified (poly)peptide as a fusion protein with a C-terminal tag of the amino acid sequence C(X)ₙ, wherein n is an integer of 1 to 100, and X is any amino acid with the exception of C, reacting said fusion protein with 2-nitro-5-thiocyanatobenzoic acid or salt thereof under conditions that allow to cyanylate the cysteine residue of the C-terminal tag, and contacting the cyanylated fusion protein with a nucleophile selected from the group consisting of ammonia, an ammonium salt or a primary amino group containing agent under conditions that allow cleaving the cyanylated fusion protein and obtain the C-terminally modified (poly)peptide of interest. The invention also encompasses artificial (poly)peptides that comprise a (poly)peptide of interest and the amino acid sequence C(X)ₙ fused to its C-terminus.

## Description

### FIELD OF THE INVENTION

The present invention is in the field of molecular biology and (poly)peptide synthesis and relates to methods for the C-terminal modification of a (poly)peptide including cyanylation with 2-nitro-5-thiocyanatobenzoic acid or salt thereof and subsequent cleavage using a nucleophile.

### BACKGROUND OF THE INVENTION

To date, recombinant protein/enzyme production for use in industrial processes is widely established. It is expected that in the future more and more industrial processes that are currently based on traditional chemistry will be adapted to involve recombinant technologies.

Recent years have seen an increasing demand for (poly)peptides and proteins for various purposes, including pharmaceuticals. Many of these peptides, polypeptides, and proteins are produced recombinantly by expression in a host organism and subsequent isolation. While said production method provides for many advantages, it is notoriously difficult to produce peptides that are modified in that they have non-naturally occurring modifications or modifications that cannot be made by the producing host organism. These modifications include C-terminal modifications, such as amidation or the addition of certain tags. While such modifications may be added by chemical synthesis, many of the (poly)peptides and proteins are susceptible to degradation or denaturation under the reaction conditions required for such modification.

There is thus need in the art for the provision of methods that allow the easy modification of (poly)peptides and proteins without adversely affecting their stability, integrity or three-dimensional conformation. The present methods solve this need by providing a method for the simply and efficient C-terminal modification of (poly)peptides and proteins with a wide range of nucleophilic agents while allowing easy separation and purification of the obtained modified (poly)peptides.

### SUMMARY OF THE INVENTION

In a first aspect, the present invention concerns a method for the C-terminal modification of a (poly)peptide of interest, wherein said (poly)peptide of interest is free of cysteine residues and at least 2 amino acids in length, said method comprising:
a) providing a fusion protein comprising the (poly)peptide of interest with the amino acid sequence C(X)ₙ fused to its C-terminus, wherein n is an integer of 1 to 100, and X is any amino acid with the exception of C, and wherein preferably the fusion protein is an artificial protein;
b) reacting the fusion protein with 2-nitro-5-thiocyanatobenzoic acid (NTCB) or salt thereof to cyanylate the cysteine residue; and
c) contacting the cyanylated fusion protein with a nucleophile selected from the group consisting of ammonia, an ammonium salt or a primary amino group containing agent to cleave the cyanylated fusion protein and obtain the C-terminally modified (poly)peptide of interest.

In various embodiments of these methods steps b) and/or c), preferably both, are carried out at a pH of 8.0-10.0, preferably at pH 9.0.

In various embodiments, steps b) and c) are carried out simultaneously, preferably by adding NTCB and the nucleophile at the same time. Alternatively, steps b) and c) can be carried out subsequently.

In step c) ammonia or an ammonium salt, such as ammonium hydroxide or ammonium acetate, may be used, typically in form of an aqueous solution or an ammonia-containing buffer. In such embodiments, the C-terminal modification is an amidation of the terminal carboxy group. According to the invention, n is at least 1, preferably n is 1 to 20 or 1 to 17 or 1 to 12 or 1 to 10 or 1 to 6 or 1 to 2 or n is 1; and/or n is selected such that the amino acid sequence C(X)ₙ has a length that is at least 5% of the total length of the (poly)peptide of interest.

In various other embodiments, in step c) a monomeric amino acid or a peptide of 2 to 100 amino acids, preferably 2 to 50 or 2 to 25 or 2 to 15 amino acids, in length is used as the nucleophile, in particular as the amino group containing agent. In such embodiments, the C-terminal modification is a C-terminal elongation by said amino acid or peptide. In various embodiments, said amino acid is not C or said peptide used as the nucleophile does not contain a C residue, in particular in instances where steps b) and c) are carried out simultaneously. In various embodiments, said monomeric amino acid is not G. In such embodiments, n may be 1 or n is selected such that the C-terminal tag consisting of the amino acid sequence C(X)ₙ differ in length by at least 1, preferably by at least 2 amino acids. In various embodiments, said difference in length is at least 5% of the total length of the (poly)peptide of interest.

In various embodiments of the methods disclosed herein, the (poly)peptide of interest is 4 or 5 to 1000 amino acids in length, preferably 5 or 10 to 500 amino acids or 15 to 250 amino acids or 20 to 150 amino acids or 5 to 100 amino acids or 4 to 50 amino acids.

In various embodiments, the method may further comprise the step of purifying or enriching the C-terminally modified (poly)peptide of interest, preferably relative to the non-modified (poly)peptide of interest. Purification or enrichment may be carried out by chromatography, preferably size exclusion chromatography, affinity chromatography or HPLC.

In various embodiments, the fusion protein and the C-terminally modified (poly)peptide of interest obtained in step c) differ in length by at least 1 amino acid, preferably by at least 2 amino acids, preferably the fusion protein differs from the C-terminally modified (poly)peptide of interest in molecular mass by at least 2.5%, preferably by at least 5%.

In various embodiments, the fusion protein is recombinantly produced in a host organism.

In various embodiments, the fusion protein further comprises an amino acid sequence derived from an RTX protein, preferably derived from HlyA, that preferably facilitates or increases renaturation of the (poly)peptide of interest, fused to the N-terminus of the (poly)peptide of interest.

In various embodiments, the fusion protein is an artificial, non-natural protein in that the C-terminal tag does not naturally occur in combination with the (poly)peptide of interest. In various embodiments, this means that the fusion protein differs from any naturally occurring polypeptide or fragment thereof by its amino acid sequence in at least one position. This particularly applies if the (poly)peptide of interest is a naturally occurring (poly)peptide or fragment thereof. In such instances, the fusion protein differs from the naturally occurring (poly)peptide of interest in that it comprises the artificial peptide tag C(X)ₙ on its C-terminus that differs from the C-terminus of the naturally occurring (poly)peptide.

If the fusion protein does comprise an amino acid sequence derived from an RTX protein, preferably derived from HlyA, that preferably facilitates renaturation of the (poly)peptide of interest fused to the N-terminus of the (poly)peptide of interest, said part of the fusion protein may also be artificially added in that said part and the (poly)peptide of interest do not naturally occur in combination. In such instances, the fusion protein may also be an artificial fusion protein in that the N-terminal RTX-derived sequence, the (poly)peptide of interest, and the C-terminal C(X)ₙ sequence do not naturally occur in combination, i.e. differ in at least one amino acid position from any naturally occurring (poly)peptide or fragment thereof.

In various embodiments, the (poly)peptide of interest has biological activity, preferably in a mammal, more preferably in a human, or is useful as a pharmaceutical for a mammal, preferably a human.

In another aspect, the present invention also relates to an isolated (poly)peptide comprising a (poly)peptide of interest and the amino acid sequence C(X)ₙ fused to its C-terminus, wherein n is an integer of 1 to 100, and X is any amino acid with the exception of C, wherein said (poly)peptide of interest and said amino acid sequence C(X)ₙ are artificially combined. In various embodiments, the isolated (poly)peptide further comprises an amino acid sequence derived from an RTX protein, preferably derived from HlyA, that facilitates or increases renaturation of the (poly)peptide of interest fused to the N-terminus of the (poly)peptide of interest.

### DETAILED DESCRIPTION OF THE INVENTION

The terms used herein have, unless explicitly stated otherwise, the meanings as commonly understood in the art.

"At least one", as used herein, relates to one or more, in particular 1, 2, 3, 4, 5, 6, 7, 8, 9, 10 or more. "At least" if used in relation to a numerical value and in particular a list of numerical values relates to each of said separate numerical values and defines said numerical value as the minimum value. "At least 1, 2, 3, etc." thus means at least 1, at least 2, at least 3 and so on.

"Isolated" as used herein in relation to a molecule means that said molecule has been at least partially separated from other molecules it naturally associates with or other cellular components. "Isolated" may mean that the molecule has been purified to separate it from other molecules and components, such as other proteins and nucleic acids and cellular debris, in particular those that accompany it due to its recombinant production in host cells.

"Nucleic acid" as used herein includes all natural forms of nucleic acids, such as DNA and RNA. Preferably, the nucleic acid molecules of the invention are DNA.

The term "peptide" is used throughout the specification to designate a polymer of amino acid residues connected to each other by peptide bonds. A peptide according to the present invention may have 2-100 amino acid residues. The terms "protein" and "polypeptide" are used interchangeably throughout the specification to designate a polymer of amino acid residues connected to each other by peptide bonds. A protein or polypeptide according to the present invention has preferably more than 100 amino acid residues. The term "(poly)peptide" is used herein to cover both, "peptides" and "polypeptides", as defined herein above.

The term "fusion protein" as used herein concerns two or more peptides and proteins which are N- or C-terminally connected to each other, typically by peptide bond(s), including via an amino acid/peptide linker sequence. Such fusion proteins may be encoded by two or more nucleic acid sequences which are operably fused to each other.

"Renaturation" and "renaturation efficiency", as used herein in relation to the (poly)peptides of the invention, primarily relates to the possibility to isolate the polypeptide in denatured/unfolded form, for example from insoluble protein aggregates, such as inclusion bodies, and induce its proper folding to the active, three-dimensional conformation. The efficiency may be given as the mass of successfully renatured protein relative to the total mass of all expressed protein, including insoluble protein, in percent or the solubilized protein mass in percent. The refolding may be effected in special refolding buffers including earth alkaline metal ions, in particular calcium ions, to induce refolding of the first amino acid sequence, which in turn facilitates renaturation of the complete fusion construct into its functional conformation. "Renaturing" and "refolding" are used interchangeably herein and generally relate to the process of folding a given protein that is unfolded or denatured such that it adopts its functional three-dimensional conformation.

Generally, the skilled person understands that for putting the present invention into practice any nucleotide sequence described herein may comprise an additional start and/or stop codon or that a start and/or stop codon included in any of the sequences described herein may be deleted, depending on the nucleic acid construct used. The skilled person will base this decision, e.g., on whether a nucleic acid sequence comprised in the nucleic acid molecule of the present invention is to be translated and/or is to be translated as a fusion protein. In various embodiments, the (poly)peptides of the invention, such as the fusion proteins or the (poly)peptides of interest, additionally comprise the amino acid M on the N-terminus.

The present invention is based on the surprising finding that cysteine-free (poly)peptides may be C-terminally modified by first adding a cysteine-containing tag to their C-terminus, wherein the cysteine residue is directly linked to the C-terminal amino acid of the (poly)peptide by a peptide bond, cyanylation of the cysteine residue by reaction with 2-nitro-5-thiocyanatobenzoic acid (NTCB) or salt thereof and subsequent cleavage by a nucleophile of choice, i.e. ammonia, ammonium or a compound comprising a primary amino group, that provides for the desired modification.

Described herein is thus a method for the C-terminal modification of a (poly)peptide of interest, wherein said (poly)peptide of interest is free of cysteine residues and at least 2 amino acids in length, said method comprising:
a) providing the (poly)peptide of interest in form of a fusion protein with the amino acid sequence C(X)ₙ fused to its C-terminus, wherein n is an integer of 1 to 100, and X is any amino acid with the exception of C;
b) reacting the fusion protein with 2-nitro-5-thiocyanatobenzoic acid (NTCB) or salt thereof to cyanylate the cysteine residue; and
c) contacting the cyanylated fusion protein with a nucleophile selected from the group consisting of ammonia, an ammonium salt or a primary amino group containing agent to cleave the cyanylated fusion protein and obtain the C-terminally modified (poly)peptide of interest.

It is important that the (poly)peptide of interest does not contain any cysteine residues since the presence of cysteine residues in its primary sequence will result in cleavage of the (poly)peptide of interest at said position by the reaction with NTCB which non-specifically cleaves the peptide bond N-terminal to any cysteine residue. Thus, if the (poly)peptide of interest does include such cysteine residues it may be mutated or modified to remove said cysteine residues before subjecting it to the reaction with NTCB.

If reference is made herein to "NTCB" or "2-nitro-5-thiocyanatobenzoic acid", both terms being used interchangeably herein, said term is intended to mean the free acid as well as any salt thereof.

To modify the C-terminus of the (poly)peptide of interest, it is first provided in form of a fusion protein with a cysteine residue directly attached to its C-terminus by a common peptide bond. Said cysteine residue is the N-terminal residue of a peptide (X)ₙ, as defined herein. In various preferred embodiments, said peptide (including the N-terminal cysteine) comprises 2 to 50 amino acids, preferably 2, 3, 4, 5, 6, 7, 8, 9, 10, 11, 12, 13, 14, 15, 16, 17, 18, 19, or 20 amino acids. The cysteine-residue containing peptide is thus artificially fused to the (poly)peptide of interest to provide for the necessary modification site that reacts with NTCB and provides for the desired C-terminal modification of the (poly)peptide of interest.

The amino acid sequence C(X)ₙ and the (poly)peptide of interest are thus artificially combined and form an artificial fusion protein. This "artificially combined" means that the respective combination of the two sequences does not occur in nature in that the (poly)peptide of interest does not comprise a cysteine residue or said cysteine-containing peptide in this position in its natural form, i.e. is a non-natural or "artificial" fusion protein. This may also mean that the (poly)peptide of interest and the amino acid sequence C(X)ₙ are heterologous to each other, i.e. they do not occur naturally in combination in any natural organism. This also applies if the (poly)peptide of interest itself is a fragment of a naturally occurring (poly)peptide, i.e. also in these cases the fragment does not comprise the amino acid sequence C(X)ₙ as its natural C-terminal flanking sequence. The fusion protein is thus an artificial fusion protein in the sense that its sequence consisting of the (poly)peptide of interest and the amino acid sequence C(X)ₙ are not identical to any naturally occurring (poly)peptide or fragment thereof, i.e. differ from such naturally occurring (poly)peptides or fragments thereof by at least one amino acid. In various embodiments, at least the C residue artificially introduced and preferably also at least one of the amino acids C-terminal to the C residue do not occur in the corresponding positions of any naturally occurring (poly)peptide or fragment thereof that has sequence similarity or identity with the (poly)peptide of interest part of the fusion protein.

"Non-natural", as used herein in relation to the fusion protein, means that said combined sequence of the (poly)peptide of interest and the C-terminal tag sequence C(X)ₙ does not naturally occur in an organism, but has been obtained by protein/peptide engineering. In other words, the fusion protein is in its totality not identical to a naturally occurring protein or fragment thereof. The term "non-natural" also means that the fusion protein is not a fragment of a naturally occurring polypeptide but rather differs from any such fragment of a naturally occurring polypeptide in sequence. The sequence identity of the fusion protein over its entire length is thus not 100% identical relative to any naturally occurring protein, polypeptide or fragment thereof, but always differs in at least 1 amino acid position.

"Heterologous", as used herein in relation to amino acid sequences, means that the respective amino acid sequences do not occur in combination in a single natural or naturally occurring protein or polypeptide or fragment thereof. This means that these two sequence elements do not occur in the same linear polypeptide sequence of a known natural polypeptide.

The C-terminal modification means a modification of the C-terminal carboxyl group. In various embodiments, said modification is an amidation, i.e. the C-terminal carboxyl group of the main peptide chain is amidated, i.e. converted into the group -C(=O)-NH₂. The reaction comprises two steps, first a cyanylation of the cysteine side chain and subsequently the cleavage of the peptide bond between the cysteine residue and the amino acid directly N-terminally to it by use of a nucleophile, i.e. a primary amino group containing agent or ammonia/ammonium. Said cleavage reaction leaves the cyclized cysteine residue or a peptide with the cyclized cysteine residue on its N-terminus and the desired C-terminally modified, for example amidated, peptide consisting of the peptide N-terminal to the cysteine residue. Said reaction is schematically shown in the scheme below, where R¹ and R² are amino acid side chains and R³ is either H or the rest of an amino acid or peptide. The first reaction step is the cyanylation of the cysteine residue by NTCB, which is converted into thionitrobenzoate (TNB) by said reaction. The cleavage occurs by action of a nucleophile, here R³-NH₂, which results in cleavage of the peptide bond and the desired C-terminally modified peptide (the N-terminal part of the starting (poly)peptide) up to the cysteine residue) and a 2-iminothiazolidine-4-carboxylyl (Itz) peptide. In an undesired side reaction, the peptide is not cleaved but a beta elimination converts the cysteine residue into dehydroalanine.

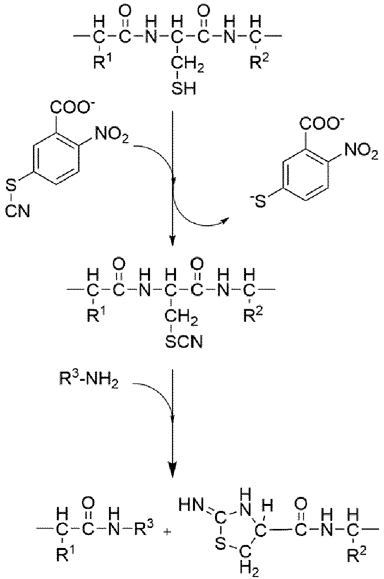

It is important to note that since only reduced cysteine residues are cyanylated, it may be necessary to provide the fusion protein for the reaction under reducing conditions. Suitable reducing agents and reducing buffers are well known in the art and can be routinely chosen by those skilled in the art. Suitable agents include, without limitation, dithiothreitol, beta-mercaptoethanol, and tris(2-carboxyethyl)phosphine (TCEP) and its hydrochloride salt. In particular the latter may be preferred in various embodiments and may be used in amounts of 1 to 25 mM, such as about 5 mM.

The fusion protein may be provided in a suitable buffer. Typically, to ensure accessibility of the cysteine residue for the reaction with NTCB, denaturing buffers may be used, including those containing urea or guanidinium chloride. In various embodiments, buffers with 4 to 12 M urea, such as about 8 M urea, or 4 to 10 M guanidinium chloride, such as about 6 M, may be used. In other embodiments, buffers that comprise urea in combination with other buffers salts, such as HEPES, may be used, for example buffers with about 400 mM urea and about 20 mM HEPES. In still other embodiments, buffers with guanidinium chloride and other components, such as salts, may be used, including NaCl, CaCl₂, and EDTA. One such exemplary buffer may comprise 300 mM guanidinium chloride, 150 mM NaCl, 10 mM CaCl₂, and 0.5 mM EDTA.

In various embodiments, the fusion protein can be provided in an ammonia-containing buffer comprising NH₄OH or NHaOAc. In various embodiments, the buffer contains NH₄OAc, preferably in a concentration of 500 mM to 1.2 M NHaOAc, such as 1 M or 800 mM. In such an embodiment, no further nucleophile has to be added to the reaction mixture for cleavage reaction, and cyanylation with NTCB and subsequent cleavage reaction can be carried out in a one-step reaction. Such procedure yields the amidated (poly)peptide of interest.

The fusion protein may be recombinantly produced, for example by expression in a suitable host organism, and may be isolated and/or purified prior to the cyanylation reaction.

The cyanylation reaction with NTCB is typically carried out under alkaline conditions, i.e., a pH of above 7.0, typically above 7.5. It is preferred that the pH ranges between 7.5 and 11.0, preferably between 8.0 and 10.0, more preferably about 9.0. The same pH conditions may also be used in step c), the cleavage step. The pH may be adjusted by a suitable base. In case, the modification is an amidation, ammonia may be used as the base and the nucleophile. In various other embodiments, the nucleophile may be the base or may be used in combination with a base, for example any hydroxide, such as an alkali metal hydroxide. In various embodiments, an ammonia-containing buffer, preferably NHaOAc, is used. In such an embodiment, the pH should be adjusted to pH 8 to pH 10, preferably to pH 9, e.g., by adding aqueous NH₃ as the base.

In the cyanylation step b), NTCB may be used in sufficient molar excess to allow a quantitative cyanylation of the cysteine residues in the fusion protein. The amount may thus range from about 20-to 100-fold molar excess of NTCB relative to the moles of all cysteines in the fusion protein or the totality of the proteins/peptides in the sample, preferably a molar excess of 25- to 75-fold, such as 30- to 70-fold, 40- to 60-fold or about 50-fold. The NTCB may be added in solid form to the sample, typically an aqueous buffer solution containing the fusion protein and optionally a reducing agent, such as TCEP. The pH may be adjusted with a suitable base and/or acid afterwards, such as a hydroxide or an inorganic or organic acid, such as acetic acid. If the cyanylation step b) is carried out simultaneously with step c), the cleavage, the added base may also be the nucleophile, such as the ammonia or the primary amino group containing compound. In various embodiments, an ammonia-containing buffer can be used that acts as base and nucleophile. Preferably, the ammonia-containing buffer contains the fusion protein and optionally a reducing agent, such as TCEP, and subsequently, NTCB is added, preferably together with a denaturation agent, such as guanidine HCl (GuHCl), to start the cyanylation and the subsequent cleavage reaction.

The cyanylation reaction is typically carried out for about 10 minutes to 3 hours, typically about 30 minutes to 2 hours, preferably about 45 minutes to about 1.5 hours, such as about 1 h at a temperature between 20 and 40°C, typically about 37°C. If the cleavage reaction is carried out simultaneously, the same incubation conditions apply for both cyanylation and cleavage.

If the cleavage reaction is carried out separately, the sample may be purified after the cyanylation, for example by subjecting it to a desalting step to remove residual NTCB and generated TNB. Afterwards, the pH may be adjusted again and the nucleophile added. The cleavage reaction may also be carried out at a temperature of 20 to 40°C, typically at about 37°C, for up to 12 hours, typically 0.5 to 2 h. However, reaction times may be adapted depending on the completeness of the cleavage reaction.

If cyanylation and cleavage are conducted simultaneously in a single sample, after the reaction is completed, residual NTCB and TNB may be removed by desalting the sample, for example by chromatographic methods or dialysis.

The length of the amino acid sequence C(X)ₙ, i.e., the choice of n, may depend on the length of the (poly)peptide of choice. As the modified polypeptide needs to be separated from non-modified fusion protein, it is generally advantageous if the difference in mass between the fusion protein and the cleaved and modified (poly)peptide of choice is enough to allow separation, for example by chromatography, such as size exclusion chromatography, or gel electrophoresis. Accordingly, if the C-terminal modification is an amidation of the terminal carboxy group, i.e. no amino acids are added to the C-terminus, n is at least 1, optionally at least 2, 3, 4, 5, 6, 7, 8, 9, 10, 11, 12, 13, 14, or 15. It is understood that the longer the fusion tag, i.e. the greater n is, the easier the separation of the modified (poly)peptide of interest and the non-cleaved fusion protein will be. However, if the (poly)peptide of interest is comparably short, for example only 4 or 15 amino acids in length, n being 1 to 5 may already be sufficient to allow easy separation. To the contrary, if the (poly)peptide of interest is comparably long, for example more than 100 or more than 200 amino acids in length, it may be advantageous if n is at least 5 or at least 10. In various embodiments, n may be chosen such as the length of the total amino acid sequence C(X)ₙ corresponds to at least 1%, 2%, 3%, 4%, 5%, 6%, 7%, 8%, 9% or 10% of the total length of the (poly)peptide of interest. This may mean, for example, that if the (poly)peptide of interest is 200 amino acids in length, the tag length may be 2, 4, 6, 8, 10, 12, 14, 16, 18 or 20 or more amino acids. Furthermore, as terminal cysteine residues are susceptible to degradation or modification during recombinant expression, n is 1 or more, i.e. the cysteine is not the C-terminal amino acid of the fusion protein.

In various embodiments, the minimum for n is 1 and the maximum is 100. Typically, n ranges between 1 and 50, for example between 1 and 30, or 1 and 25, or 2 to 20, 3 to 15. In various embodiments, n may be 0, 1, 2, 3, 4, 5, 6, 7, 8, 9, 10, 11, 12, 13, 14, 15, 16, 17, 18, 19 or 20.

While the amino acid sequence of (X)ₙ is not particularly limited, it has surprisingly been found by the inventors that certain amino acids C-terminal to the C residue advantageously influence the cleavage reaction. In various embodiments, the amino acid sequence (X)ₙ is therefore X¹(X)ₙ₋₁, wherein X¹ is not W, or is not W, Y, F, or P or is no bulky amino acid, such as W, Y, F. In various embodiments, X¹ may be an unpolar amino acid, such as A, V, L or I, preferably L, a basic amino acid, such as K or R, preferably K, or an acidic amino acid, such as D or E. In various embodiments, X¹(X)ₙ₋₁ is L, LW, K, or E. In various embodiments, X¹(X)ₙ₋₁ is not 6x-His. In various embodiments, X¹(X)ₙ₋₁ is not PNDKYE (SEQ ID NO:5). These amino acid sequence of (X)ₙ have been found to work particularly well for short peptides that are only up to 50 amino acids in length. In various other embodiments, in particular in embodiments wherein the (poly)peptide of interest is at least 30 or at least 50 amino acids in length, such as 51 amino acids and more, X¹(X)ₙ₋₁ may be CG₄SG₄-His₄₋₆.

In various preferred embodiments, the nucleophile is ammonia or ammonium (which is converted at alkaline pH into ammonia) and the C-terminal modification is an amidation of the terminal main chain carboxyl group.

In various other embodiments, in step c) the nucleophile is a monomeric amino acid or a peptide of 2 to 100 amino acids, preferably 2 to 50 or 2 to 25 or 2 to 15 amino acids in length. The length of said peptide may be 2, 3, 4, 5, 6, 7, 8, 9, 10, 11, 12, 13, 14, or 15 amino acids or more. Said amino acid or peptide may be or comprise any of the natural, proteinogenic amino acids with the exception of C, i.e. may be or comprise G, A, V, L, I, F, M, P, S, T, H, K, R, E, D, N, Q, W, and/or Y and/or may be any non-proteinogenic or non-natural amino acid, including but not limited to, beta-alanine, GABA, dehydroalanine, norvaline, norleucine, ornithine, allothreonine, homoserine, isovaline, sarcosine, and the like. In various embodiments, the monomeric amino acid is not G. If the nucleophile is a peptide, it may comprise any of the natural, proteinogenic amino acids with the exception of C, i.e. may comprise G, A, V, L, I, F, M, P, S, T, H, K, R, E, D, N, Q, W, and/or Y and/or may comprise any non-proteinogenic or non-natural amino acid, including but not limited to, beta-alanine, GABA, dehydroalanine, norvaline, norleucine, ornithine, allothreonine, homoserine, isovaline, sarcosine, aminoisobutyric acid and the like.

The presently claimed methods thus allow to modify a given (poly)peptide of interest with non-natural/non-proteinogenic amino acids, or peptides containing such non-natural/non-proteinogenic amino acids at its C-terminus.

The presently claimed methods also allow to modify a given (poly)peptide of interest with any compound that comprises a nucleophilic group as defined herein that facilitates cleavage. Such compounds may include, without limitation, fluorophores, pharmaceutically actives compounds, tags, such as biotin, and linker compounds, such as those having an alkyne or azide group.

In all the afore-mentioned embodiments, the amino acid or the peptide used provides the primary amino group used as a nucleophile for the cleavage reaction and for creating the new peptide bond with the C-terminal carboxyl group of the (poly)peptide of interest. In order to avoid side reactions, in some embodiments, the amino acid or peptide used as the nucleophile does not comprise any other primary amino group than the main chain N-terminal amino group, for example a side chain primary amino group.

Accordingly, in various embodiments, the amino acid is not K, N or Q or the peptide does not contain any of these amino acids.

In such embodiments, in which the C-terminal modification is a C-terminal elongation by an amino acid or peptide, n may be selected such that the C-terminal tag consisting of the amino acid sequence C(X)ₙ differs in length by at least 1, preferably by at least 2 amino acids from the amino acid or peptide used as the nucleophile. In various embodiments, n may be chosen such as the difference in length between the total amino acid sequence C(X)ₙ and the amino acid or peptide used as the nucleophile is at least 1%, 2%, 3%, 4%, 5%, 6%, 7%, 8%, 9% or 10% of the total length of the (poly)peptide of interest. This may mean that in case the nucleophile is a peptide of, for example, at least 5 amino acids in length, n may be 1, or if the nucleophile is a single amino acid or a short peptide of up to 5 amino acids, n may be 10 or more.

In various embodiments, the fusion protein and the C-terminally modified (poly)peptide of interest obtained in step c) differ in length by at least 1 amino acid, preferably by at least 2 amino acids, preferably the fusion protein differs from the C-terminally modified (poly)peptide of interest in molecular mass by at least 1%, 2%, 2.5%, 3%, 4%, preferably by at least 5%, 6%, 7%, 8%, 9%, 10% or more.

While it is generally preferred to carry out steps b) and c) simultaneously, said simultaneous reaction precludes the use of cysteine or cysteine-containing peptides as the nucleophile, since these would react with the NTCB. However, if these steps are carried out separately and residual NTCB is removed prior to the cleavage, cysteine or cysteine-containing peptides may be used as the nucleophile.

In step c), the nucleophile is used in molar excess relative to the fusion protein. The molar excess may be at least 100-fold, at least 250-fold, at least 500-fold or at least 1000-fold.

In various embodiments, the method may further comprise a step d) of purifying or enriching the C-terminally modified (poly)peptide of interest, preferably relative to the non-modified (poly)peptide of interest or the fusion protein. Such purification or enrichment may be carried out by chromatography, preferably size exclusion chromatography, affinity chromatography or HPLC, or gel electrophoresis.

The (poly)peptide of interest may be 4 or 5 to 1500 amino acids in length, preferably 10, 11, 12, 13, 14, 15, 16, 17, 18, 19, 20, 21, 22, 23, 24 or 25 to 1000, to 800, to 700, to 600, to 500, to 400, to 300, to 200, to 180, to 150, to 120 or to 100 amino acids in length. "Peptides of interest" are typically oligopeptides with a length of up to 100 amino acids, typically 3 to 80, 4 to 75, 5 to 70, 6 to 70, 7 to 70, 8 to 70, 9 to 70, or 10 to 70 amino acids. The lower limit may be 10, 11, 12, 13, 14, 15 ,16, 17, 18, 19 or 20 amino acids and the upper limit may be 70, 65, 60, 55, or 50 amino acids. "Polypeptides/proteins of interest" typically comprise more than 100 amino acids, with the upper limit as defined above, such as 110, 120, 130, 140, 150, 160, 170, 180, 190, 200, 210, 220, 230, 240, 250, 275, 300, 325, 350, 375, 400, 450, 500, 550, 600, 650, 700, 750, 800, 850, 900, 950, 1000, 1100, 1200, 1300, 1400, or 1500 amino acids. The terms polypeptide of interest and protein of interest are used interchangeably herein.

In various embodiments, the (poly)peptide of interest is a naturally occurring peptide or polypeptide sequence or fragment thereof, typically not containing any sequence deviation over its entire sequence relative to its natural template. This may mean that the (poly)peptide of interest shares 100% sequence identity over its entire length to a naturally occurring peptide or polypeptide or fragment thereof.

In various embodiments, the (poly)peptide of interest may comprise two or more naturally occurring peptides or proteins, wherein the two or more peptides or proteins may be separated by protease cleavage sites. This also includes embodiments, where the same peptide or protein is included multiple times in said (poly)peptide of interest. This then allows production of higher amounts of the respective (poly)peptide of interest. In other embodiments, the (poly)peptide of interest is only a single peptide or protein.

Generally, any peptide or protein may be chosen as (poly)peptide of interest.

In various embodiments, the (poly)peptide of interest has biological activity, preferably in a mammal, more preferably in a human, or is useful as a pharmaceutical for a mammal, preferably a human. "Biological activity", as used in this context, refers to the property that the (poly)peptide interacts specifically with a target molecule within a target organism and that said interaction is specific (in that it occurs favorably over other interactions) and causes a biologic response in the target organism. In other words, biological activity is the ability of a particular molecular entity to bring about a specific biological change on the target that is typically measurable or determinable. Said biological activity may include the property to function to prevent, treat or alleviate the symptoms of a disease or disorder.

In certain embodiments, the protein of interest is a protein, which does not form a homodimer or homo-multimer. The (poly)peptide of interest may also be a peptide or protein which is a subunit of a larger peptide or protein complex. Such a peptide or protein may be isolated after expression and be suitable for an *in vitro* reconstitution of the multi peptide or protein complex.

In certain embodiments, the (poly)peptide of interest is a protein or peptide having less than 1000, less than 800, less than 700, less than 600, less than 500, less than 400, less than 300 amino acid residues, for example less than 200 amino acids or less than 150 amino acids. If these peptides comprise pre-and/or pro-sequences in their native state after translation the nucleic acid sequence encoding for the (poly)peptide of interest may be engineered to be limited to the sequence encoding the mature (poly)peptide. One exemplary peptide is insulin, e.g., human insulin. The expression of over-expressed peptides and proteins as inclusion bodies is especially advantageous where the peptide or protein is harmful to the host cell.

In various embodiments, the (poly)peptide of interest is an enzyme. The International Union of Biochemistry and Molecular Biology has developed a nomenclature for enzymes, the EC numbers; each enzyme is described by a sequence of four numbers preceded by "EC". The first number broadly classifies the enzyme based on its mechanism. The complete nomenclature can be browsed at http://www.chem.qmul.ac.uk/iubmb/enzyme/.

Accordingly, a (poly)peptide of interest according to the present invention may be chosen from any of the classes EC 1 (Oxidoreductases), EC 2 (Transferases), EC 3 (Hydrolases), EC 4 (Lyases), EC 5 (Isomerases), and EC 6 (Ligases), and the subclasses thereof.

In certain embodiments, the (poly)peptide of interest is cofactor dependent or harbors a prosthetic group. For expression of such peptides or proteins, in some embodiments, the corresponding cofactor or prosthetic group may be added to the culture medium during expression.

In certain cases, the (poly)peptide of interest is a dehydrogenase or an oxidase. In case the (poly)peptide of interest is a dehydrogenase, in some embodiments, the (poly)peptide of interest is chosen from the group consisting of alcohol dehydrogenases, glutamate dehydrogenases, lactate dehydrogenases, cellobiose dehydrogenases, formate dehydrogenases, and aldehydes dehydrogenases. In case the (poly)peptide of interest is an oxidase, in some embodiments, the (poly)peptide of interest is chosen from the group consisting of cytochrome P450 oxidoreductases, in particular P450 BM3 and mutants thereof, peroxidases, monooxygenases, hydrogenases, monoamine oxidases, aldehydes oxidases, xanthin oxidases, amino acid oxidases, and NADH oxidases.

In further embodiments, the (poly)peptide of interest is a transaminase or a kinase. In case the (poly)peptide of interest is a transaminase, in some embodiments, the (poly)peptide of interest is chosen from the group consisting of alanine aminotransferases, aspartate aminotransferases, glutamate-oxaloacetic transaminases, histidinol-phosphate transaminases, and histidinol-pyruvate transaminases. In various embodiments, if the (poly)peptide of interest is a kinase, the (poly)peptide of interest is chosen from the group consisting of nucleoside diphosphate kinases, nucleoside monophosphate kinases, pyruvate kinase, and glucokinases. In some embodiments, if the (poly)peptide of interest is a hydrolase, the (poly)peptide of interest is chosen from the group consisting of lipases, amylases, proteases, cellulases, nitrile hydrolases, halogenases, phospholipases, and esterases.

In certain embodiments, if the (poly)peptide of interest is a lyase, the (poly)peptide of interest is chosen from the group consisting of aldolases, e.g., hydroxynitrile lyases, thiamine-dependent enzymes, e.g., benzaldehyde lyases, and pyruvate decarboxylases. In various embodiments, if the (poly)peptide of interest is an isomerase, the (poly)peptide of interest is chosen from the group consisting of isomerases and mutases.

In some embodiments, if the (poly)peptide of interest is a ligase, the (poly)peptide of interest may be a DNA ligase.

In certain embodiments, the (poly)peptide of interest may be an antibody. This may include a complete immunoglobulin or fragment thereof, which immunoglobulins include the various classes and isotypes, such as IgA, IgD, IgE, IgGI, IgG2a, IgG2b and IgG3, IgM, etc. Fragments thereof may include Fab, Fv and F(ab')2, Fab', the variable domain of the light chain (VL) or the variable domain of the heavy chain (VH) and related fragments, such as nanobodies, and the like.

Also contemplated herein are therapeutically active peptides and proteins of interest, e.g., cytokines.

Thus, in certain embodiments the (poly)peptide of interest is selected from the group consisting cytokines, in particular human or murine interferons, interleukins (IL-1, IL-2, IL-3, IL-4; IL-5; IL-6; IL-7; IL-8; IL-9; IL-10; IL-11; IL-12; IL-13; IL-14; IL-15; IL-16; and IL-17), colony-stimulating factors, necrosis factors, e.g., tumor necrosis factor, such as TNF alpha, and growth factors, such as transforming growth factor beta family members, such as TGF-beta1; TGF-beta2 and TGF-beta3.

In some embodiments, if the (poly)peptide of interest is an interferon, the (poly)peptide of interest may be selected from the group consisting of interferon alpha, e.g., alpha-1, alpha-2, alpha-2a, and alpha-2b, alpha-2, alpha-16, alpha 21, beta, e.g., beta-1, beta-1a, and beta-1b, or gamma.

In further embodiments, the (poly)peptide of interest is an antimicrobial peptide, in particular a peptide selected from the group consisting of bacteriocines and lantibiotics, e.g., nisin, cathelicidins, defensins, and saposins.

Also disclosed herein are peptides or proteins of interest which are therapeutically active peptides or proteins. In certain embodiments, the (poly)peptide of interest is a therapeutically active peptide. In some embodiments, a therapeutically active peptide may be selected from the group consisting of Fuzeon/T20, human calcitonin, salmon calcitonin, human corticotropin release factor, Mab40, Mab42, peptides associated with Alzheimer's disease, exenatide, Tesamorelin, Teriparatide, BMP-2, Corticorelin ovine triflutate, Linaclotide, Nesiritide, Lucinactant, Bivalirudin, Lepirudin, Thymalfasin, Glatiramer, Glucagon, Aviptadil, Secretin, Thymosin-b4, Teduglutide, GLP-1, GLP-2 and analoga, Plecanatide, Ecallantide, Anakinra, Disiteritide, Lixisenatide, Liraglutide, Semaglutide, Abaloparatide, Goserelin, Lanreotide, Carfilzomib, Enfuvirtide, T-20, Terlipressin, Elcatonin, Afamelanotide, Oxodotreotide, Caspofungin, Colistin, Polymyxin E, Cyclosporine, Dactinomcyin, Lyovac-Cosmegen, Degarelix, Vancomycin, Secretin, Ziconotide, Gonadorelin, Somastatin, Sincalide, Eptifibatid, Vapreotide, Triptorelin, Desmopressin, Lypressin, Atosiban, Pramintide, Pasireotide, Sandostatin, and Icatibant. The afore-mentioned peptides may be of mammalian or human origin. Also encompassed are analogues of the afore-mentioned peptides that originate from other species, for example homologues from other animals, microorganisms, virus and others.

In certain embodiments, the (poly)peptide of interest is a type I secretion substrate. More than 1000 proteins are annotated or have been described as type I secretion substrates in the literature. Many of them have interesting characteristics for the biotechnological usage, in particular proteases and lipases. Suitable proteases and lipases have been described by Baumann et al. (1993) EMBO J 12, 3357-3364; and Meier et al. (2007) J. BIOL. CHEM.: 282(43), pp. 31477-31483. The content of each of these documents is incorporated by reference herein in its entirety.

In certain embodiments, the (poly)peptide of interest is chosen from the group consisting of MBP, lipase CalB, protease SprP, hydrolase PlaB, hydrolase PlaK, hydrolase PlbF, lipase TesA, Vif, human interferon alpha-1, alpha-2, alpha-8, alpha- 16, alpha-21, human interferon beta, human interferon gamma, murine interferon alpha, murine interferon gamma, IFABP, Cas2, affibody protein ZA3, nisin, corticotropin release factor, amyloid-beta peptide, exenatide, Fuzeon/T20, salmon calcitonin, Mab40, Mab42, lipase LipA, SprP, the HIV- 1 protein Vif, human calcitonin, Tesamorelin, Teriparatide, BMP-2, Corticorelin ovine triflutate, Linaclotide, Nesiritide, Lucinactant, Bivalirudin, Lepirudin, Thymalfasin, Glatiramer, Glucagon, Aviptadil, Secretin, Thymosin-b4, Teduglutide, GLP-1 and analogues thereof, GLP-2 and analoga, Plecanatide, Ecallantide, Anakinra, Disiteritide, Lixisenatide, Liraglutide, Semaglutide, Abaloparatide, Goserelin, Lanreotide, Carfilzomib, Enfuvirtide, T-20, Terlipressin, Elcatonin, Afamelanotide, Oxodotreotide, Caspofungin, Colistin, Polymyxin E, Cyclosporine, Dactinomcyin, Lyovac-Cosmegen, Degarelix, Vancomycin, Secretin, Ziconotide, Gonadorelin, Somastatin, Sincalide, Eptifibatid, Vapreotide, Triptorelin, Desmopressin, Lypressin, Atosiban, Pramintide, Pasireotide, Sandostatin, Tirzepatide, Cagrilitide, and Icatibant as well as analogues of all afore-mentioned molecules.

In various embodiments, the (poly)peptide of interest is not human spectrin or a fragment thereof and/or is not the peptide having the amino acid sequence MRSPA (SEQ ID NO:6).

The (poly)peptide of interest is provided in form of a fusion protein with the cysteine-containing amino acid sequence defined above.

In various embodiments, the fusion protein comprising the (poly)peptide of interest with the cysteine-containing amino acid sequence C(X)ₙ fused to its C-terminus may comprise a third amino acid sequence fused to its N-terminus. The fusion protein would thus, from N- to C-terminus, have the architecture:
3^{rd} amino acid sequence - (poly)peptide of interest - C(X)ₙ

Said third amino acid sequence (3^{rd} amino acid sequence) fused to the N-terminus of the (poly)peptide of interest may be a GG repeat containing sequence optionally derived from an RTX protein. Said third amino acid sequence may have a beneficial effect on expression, ease of isolation, stability, solubility, and/or renaturation of the (poly)peptide of interest (in form of the fusion protein). In various embodiments, it increases renaturation of the (poly)peptide of interest. This is advantageous, since in the course of its processing, said (poly)peptide is subjected to multiple conditions that may cause its denaturing so that renaturation may be required at some stage to refold it into its proper three-dimensional structure. The third amino acid sequence may serve to increase the efficiency of said renaturation.

The RTX proteins from which the third amino acid sequences may be derived can, in various embodiments, be allocrits of a T1 secretion system (T1 SS) and may be selected from the group consisting of, without limitation, HlyA, CyaA, EhxA, LktA, PILktA, PasA, PvxA, MmxA, LtxA, ApxlA, ApxllA, ApxlllA, ApxIVA, Apxl, Apx11, AqxA, VcRtxA, VvRtxA, MbxA, RTX cytotoxin, RtxL1, RtxL2, FrhA, LipA, TliA, PrtA, PrtSM, PrtG, PrtB, PrtC, AprA, AprX, ZapA, ZapE, Sap, HasA, colicin V, LapA, ORF, RzcA, RtxA, XF2407, XF2759, RzcA, RsaA, Crs, CsxA, CsxB, SlaA, SwmA, S111951, NodO, PlyA, PlyB, FrpA, FrpC, RTX1, RTX3, RTX7, RTX8, RTX9, RTX12, RTX14, RTX16, RTX18, RTX19, RTX20, RTX21, RTX22, RTX24, RTX26, RTX28, RTX29, RTX30, RTX31, RTX32, and RTX33. The amino acid sequences of the recited RTX proteins are available in the Uni Prot database under the following accession numbers:
RTX1 (SCP-domain containing protein, *Aromatoleum aromaticum strain EbN1*; NCBI Reference WP_011239843.1; UniProt Accession No. Q5NXL6),
RTX3 (Serralysin (*Caulobacter sp.* Strain K31); UniProt Accession No. BOT558),
RTX7 (uncharacterized protein of *Pelobacter propionicus*; UniProt Accession No. A1ARK7; NCBI Reference WP_011736233.1),
RTX8 (Metallopeptidase AprX (*Pseudomonas fluorescens strain ATCC BAA-477*/*NRRL B-23932* / *Pf-*5); UniProt Accession No. Q4KDU3; NCBI Reference WP_011060780.1),,
RTX9 (glycosyl hydrolase family 16, hemolysin-type calcium-binding repeat protein (*Rhodobacter sphaeroides strain ATCC 17023* / *DSM 158* / *JCM 6121*); UniProt Accession No. Q3J0J9; NCBI Reference WP_011338282.1),
RTX12 (endonuclease/exonuclease/phosphatase (*Caulobacter sp.* Strain K31); UniProt Accession No. B0T829; NCBI Reference WP_012287684.1),
RTX14 (Glycoside hydrolase family 16 (*Caulobacter sp.* Strain K31); UniProt Accession No. B0T438), RTX16 (lipase class 3 (*Chlorobium phaeobacteroides strain DSM266*); UniProt Accession No. A1BIU7; NCBI Reference WP_011746110.1),
RTX18 (animal haem peroxidase (*Leptotrix cholodnii srain ATCC 51168* / *LMG 8142* / *SP-6*); UniProt Accession No. B1Y442; NCBI Reference WP_012347322.1),
RTX19 (putative outer membrane adhesin like protein (*Magnetococcus marinus strain ATCC BAA-1437* /*JCM 17883* / *MC-1*); UniProt Accession No. A0L6L9; ENA Reference ABK43612.1),
RTX20 (putative outer membrane adhesin like protein (*Magnetococcus marinus strain ATCC BAA-1437* /*JCM 17883* / *MC-1*); UniProt Accession No. A0LDY6; NCBI Reference WP_011715232.1),
RTX21 (Glycerophosphoryl diester phosphodiesterase (*Methylobacterium nodulans (strain LMG 21967* / *CNCM I-2342* / *ORS 2060*); UniProt Accession No. B8ICD8; EMBL Reference ACL55526.1),
RTX22 (5' nucleotidase domain protein (*Methylobacterium sp. Strain 4-64*); UniProt Accession No. B0UQ63; NCBI Reference WP_012331605.1),
RTX24 (polyurethanase A (*Pseudomonas fluorescens strain ATCC BAA-477* / *NRRL B* 23932 / *Pf-5*); UniProt Accession No. Q4KBS6; NCBI Reference WP_011061486.1),
RTX26 (extracellular alkaline metalloprotease AprA (*Pseudomonas fluorescens strain ATCC BAA-477*/ *NRRL B-23932* / *Pf-5*); UniProt Accession No. Q4KBR8; NCBI Reference WP_011061494.1),
RTX28 (hemolysin type calcium-binding protein (*Rhodobacter sphaeroides strain ATCC 17023* / *DSM 158*/*JCM 6121);* UniProt Accession No. Q3IVG4; NCBI Reference WP_011331290.1),
RTX29 (neutral zinc metallopeptidase (*Rhodobacter sphaeroides strain ATCC 17023* / *DSM 158* / *JCM* 6121); UniProt Accession No. Q3J1D3; NCBI Reference WP_011338088.1),
RTX30 (glycoside hydrolase family 16 (*Rhodobacter sphaeroides strain ATCC 17029* / *ATH 2.4.9*); UniProt Accession No. A3PLQ2; NCBI Reference WP_011841485.1),
RTX31 (peptidase domain protein (*Sinorhizobium medicae strain WSM419*); UniProt Accession No. A6UK74; NCBI Reference WP_011970163.1),
RTX32 (Serralysin (*Sinorhizobium medicae strain WSM419*); UniProt Accession No. A6UK83; NCBI Reference WP_011970172.1), and
RTX33 (hemolysin type calcium-binding region (*Verminephrobacter eiseniae strain EF01-2*); UniProt Accession No. A1WL15; NCBI Reference WP_011810323.1).

In various embodiments, the third amino acid sequence may be derived from hemolysin A protein, preferably any one of the sequences derived from hemolysin A and being disclosed in any one of EP2768848 A1, EP2986633 A1, EP3210997 A1, EP4121444 A1, EP4121086 A1, WO2022194375 A1, WO2024056875 A1, and WO2024056874 A1. Also encompassed are all other GG-repeat containing sequences disclosed in these references as fusion partner for (poly)peptides or proteins of interest.

In various embodiments, said third amino acid sequence may have or comprise the amino acid sequence set forth in SEQ ID NO:214 of WO2024056874A1 or a variant or fragment thereof, as defined in WO 2024056874 A1 and any other of the above-referenced publications.

The hemolysin (Hly) secretion system is a protein secretion system which mostly occurs in gram-negative bacteria. This secretion system belongs to the family of type I secretion systems which transport their substrates in an ATP driven manner in a single step from the cytosol to the extracellular space without an intermediate station in the periplasm. The Hly secretion system comprises hemolysin B (HlyB) which represents an ATP-binding cassette (ABC) transporter, the membrane fusion protein hemolysin D (HlyD), and the universal outer membrane protein ToIC. The 110 kDa hemolytic toxin hemolysin A (HlyA) is a transport substrate of the Hly secretion system. On genetic level, the components necessary for hemolysin A-specific secretion are organized in an operon structure. The nucleic acid sequence encoding for hemolysin C (HlyC) also forms part of this operon but is not required for HlyA secretion through the Hly secretion system. HlyC catalyzes acylation of HlyA which renders HlyA hemolytic. HlyA is a protein which consists of 1024 amino acid residues and requires for its export via the Hly secretion system its C-terminus, comprising about 40-60 amino acids. Furthermore, HlyA is characterized in that it comprises N-terminally to the 40-60 C-terminal amino acids a domain comprising several glycine rich (GG) repeats. The Hly secretion system and its T1 SS allocrit HlyA are referred to herein as N-terminal fusion partners of the (poly)peptides of interest in order to faciliate their expression and secretion or refolding. However, it is to be understood that the invention is not limited to such constructs derived from HlyA, since similar results have also been obtained with the GG repeat-containing sequences of other RTX proteins.

"GG repeat", "GG repeat sequence" and "glycine rich repeat", as used herein interchangeably, are the characteristic of the repeats in toxin (RTX) toxin family and have the consensus sequence GgxGxDxUx, wherein each x can independently be any amino acid and U is independently a hydrophobic, large amino acid, preferably selected from F, V, A, Y, I, L and M. The glycine rich repeats bind Ca²⁺ which induces their folding. Hence, in absence of earth alkaline metal ions, in particular Ca²⁺, the domain comprising the glycine rich repeats is typically unstructured.

The third amino acid sequence may further comprise a linker sequence between the RTX-derived sequence and its connection to the (poly)peptide of interest.

In various embodiments, the linker sequence may be 1 to 50 amino acids in length, for example 1 to 30 amino acids or 5 to 20 amino acids. In various embodiments, the linker sequence may be functional in that it may provide for easy cleavage and separation of the third amino acid sequence and the (poly)peptide of interest. To facilitate such a purpose, it can comprise or consist of a protease recognition and cleavage site. The linker may also comprise both, a linker sequence that serves only as a link and a protease cleavage site. The linker sequence may be a G-rich sequence, for example 4 or 5 consecutive G residues, optionally followed by an S residue.

The term "protease (recognition and) cleavage site" refers to a peptide sequence which can be cleaved by a selected protease thus allowing the separation of peptide or protein sequences which are interconnected by a protease cleavage site. In certain embodiments the protease cleavage site is selected from the group consisting of a Factor Xa, a tobacco edge virus (TEV) protease, a enterokinase, a SUMO Express protease, an Arg-C proteinase, an Asp-N endopeptidases, an Asp-N endopeptidase + N-terminal Glu, a caspase 1, a caspase 2, a caspase 3, a caspase 4, a caspase 5, a caspase 6, a caspase 7, a caspase 8, a caspase 9, a caspase 10, a chymotrypsin-high specificity, a chymotrypsin-low specificity, a clostripain (Clostridiopeptidase B), a glutamyl endopeptidase, a granzyme B, a pepsin, a proline-endopeptidase, a proteinase K, Welqut protease, Clean Cut protease, a staphylococcal peptidase I, a Thrombin, a Trypsin, inteins, SprB or SpIA-E from *Staphylococcus aureus*, and a Thermolysin cleavage site. In various embodiments, it may be a Factor Xa, SprB or TEV protease cleavage site. It can be preferred, in some embodiments, to design the protease recognition site such that as few amino acids as possible of the recognition and cleavage site remain attached to the peptide or protein of interest. In various embodiments, a protease recognition and cleavage site is included, the site being for example a TEV protease recognition and cleavage site.

In various embodiments, the third amino acid sequence may (further) comprise at least one affinity tag.

The term "affinity tag" as used herein relates to entities which are coupled to a molecule of interest and allow enrichment of the complex between the molecule of interest and the affinity tag using an affinity tag receptor. In certain embodiments affinity tags may be selected from the group consisting of the Strep-tag^{®} or Strep-tag^{®} II, the myc-tag, the FLAG-tag, the His-tag, the small ubiquitin-like modifier (SUMO) tag, the covalent yet dissociable NorpD peptide (CYD) tag, the heavy chain of protein C (HPC) tag, the calmodulin binding peptide (CBP) tag, or the HA-tag or proteins such as Streptavidin binding protein (SBP), maltose binding protein (MBP), and glutathione-S-transferase.

In various embodiments, the third amino acid sequence comprises as the first, N-terminal amino acid the residue M. If this is not present within its sequence, it may be artificially added, if desired, in particular to facilitate expression in a host cell.

The third amino acid sequence of the present invention is, in various embodiments, 18 to 300 amino acids in length, for example 30 to 200. The lower limit may be 20, 25, 30, 35, 40, 45, 50, 55, 60, 65, 70, 75, 80, 85, 90, 95 or 100 amino acids in length. It may be preferred that the first amino acid sequence is as short as possible, as long as its beneficial influence on expression levels and/or yields is not impaired and it retains sufficiently high solubility and/or proteolytic stability. Generally, the skilled person will know how to find a balance between a sequence that is as short as possible while still providing for the desired expression levels/yields.

In various embodiments, the third amino acid sequence does not comprise a secretion signal, i.e. an amino acid sequence that facilitates secretion from the host cell into the medium. This may mean that the third amino acid sequence does not facilitate secretion of the fusion protein to a significant degree, which may mean that at least 50 % or more of the expressed fusion protein remain inside of the cell, preferably as inclusion bodies, and are not secreted to the surrounding medium. This percentage may be higher, such as 60%, 70%, 80%, 90% or more. In various embodiments, essentially all of the expressed polypeptide remains inside in the cell. In various embodiments, it is expressed as insoluble inclusion bodies.

It has been surprisingly found that a fusion protein that also comprises said RTX-derived sequence on its N-terminus allows the C-terminal modification of the (poly)peptide of interest in a first step during which the cysteine-containing tag sequence is cleaved off. As described above, this step typically involves a step of denaturing the protein to allow accessibility of the NTCB to the cysteine residue and ensure solubility at various conditions. It has now been found that the RTX-derived tag allows simple renaturation of the (poly)peptide of interest in that the renaturation of the RTX-derived part also triggers the refolding of the (poly)peptide of interest part. Once renaturation has occurred, the RTX-derived sequence may be cleaved off, typically by use of a protease, to obtain the (poly)peptide of interest.

In various embodiments, the fusion protein is also an artificial, non-natural protein in that the third amino acid sequence fused to the N-terminus of the (poly)peptide of interest is also artificially added in that said part and the (poly)peptide of interest do not naturally occur in combination.

In various embodiments, the fusion protein is recombinantly produced in a host organism, as described in more detail herein below.

In the methods described herein, the nucleic acid encoding the fusion protein as described above may be used to produce the fusion protein. The fusion protein comprises the (poly)peptide of interest and the C-terminal C(X)ₙ sequence and optionally also a N-terminal fusion to the (poly)peptide of interest as described herein. All of these amino acid sequences are typically linked by peptide bonds and expressed as a single fusion protein. To facilitate said expression, the nucleic acid molecule comprises coding sequences for each part of the fusion protein, with said nucleotide sequences being operably linked to allow expression of the single fusion protein comprising all afore-mentioned amino acid sequences. The nucleic acid molecule encoding the fusion proteins of the invention also forms part of the present invention.

The term "operably linked" in the context of nucleic acid sequences means that nucleic acid sequences are linked such that the individual nucleic acid sequences are placed in a functional relationship with each other. For instance, a promoter sequence is operably linked to a coding sequence of a heterologous gene if the promoter can initiate the transcription of the coding sequence. If two coding sequences are operably linked this means that the encoded (poly)peptide is expressed as a single linear (poly)peptide chain.

In certain embodiments, the above defined nucleic acid molecules may be comprised in a vector, for example a cloning or expression vector. Generally, the nucleic acid molecules of the invention can also be part of a vector or any other kind of cloning vehicle, including, but not limited to a plasmid, a phagemid, a phage, a baculovirus, a cosmid, or an artificial chromosome. Generally, a nucleic acid molecule disclosed in this application may be "operably linked" to a regulatory sequence (or regulatory sequences) to allow expression of this nucleic acid molecule. The respective vectors also form part of the present invention.

Such cloning vehicles can include, besides the regulatory sequences described above and a nucleic acid sequence of the present invention, replication and control sequences derived from a species compatible with the host cell that is used for expression as well as selection markers conferring a selectable phenotype on transformed or transfected cells. Large numbers of suitable cloning vectors are known in the art, and are commercially available.

The vector may be effective for prokaryotic or eukaryotic protein expression. In particular, the nucleic acid molecules may be comprised in a vector for prokaryotic protein expression. Such vector sequences are constructed such that a sequence of interest can easily be inserted using techniques well known to those skilled in the art. In certain embodiments, the vector is selected from the group consisting of a pET-vector, a pBAD-vector, a pK184-vector, a pMONO-vector, a pSELECT-vector, pSELECT-Tag-vector, a pVITRO-vector, a pVIVO-vector, a pORF-vector, a pBLAST-vector, a pUO-vector, a pDUO-vector, a pZERO-vector, a pDeNy-vector, a pDRIVE-vector, a pDRIVE-SEAP-vector, a HaloTag^{®}Fusion- vector, a pTARGET^{™}-vector, a Flexi^{®}-vector, a pDEST-vector, a pHIL-vector, a pPIC-vector, a pMET-vector, a pPink-vector, a pLP -vector, a pTOPO-vector, a pBud-vector, a pCEP-vector, a pCMV-vector, a pDisplay-vector, a pEF-vector, a pFL-vector, a pFRT-vector, a pFastBac-vector, a pGAPZ-vector, a pIZ/V5-vector, a p3S-vector, a plAR-vector, pSEC, pMS, a pSU2726-vector, a pLenti6 -vector, a pMIB-vector, a pOG-vector, a pOpti-vector, a pREP4-vector, a pRSET-vector, a p SCREEN- vector, a pSecTag-vector, a pTEFI -vector, a pTracer-vector, a pTrc-vector, a pUB6-vector, a pVAXI-vector, a pYC2-vector, a pYES2-vector, a pZeo-vector, a pcDNA-vector, a pFLAG-vector, a pTAC-vector, a pT7-vector, a gateway^{®}-vector, a pQE-vector, a pLEXY-vector, a pRNA-vector, a pPK-vector, a pUMVC-vector, a pLIVE-vector, a pCRUZ-vector, a Duet-vector, and other vectors or derivatives thereof.

The vectors may be chosen from the group consisting of high, medium and low copy vectors.

The above described vectors may be used for the transformation or transfection of a host cell in order to achieve expression of a peptide or protein which is encoded by an above described nucleic acid molecule and comprised in the vector DNA.

The host cell may be specifically chosen as a host cell capable of expressing the fusion protein. In addition or otherwise, in order to produce the isolated polypeptide of the invention, the nucleic acid coding for it can be genetically engineered for expression in a suitable system. Transformation can be performed using standard techniques (Sambrook, J. et al. (2001), Molecular Cloning: A Laboratory Manual, 3rd Ed., Cold Spring Harbor Laboratory Press, Cold Spring Harbor, NY).

Suitable host cells can be prokaryotic cells. In certain embodiments, the host cells are selected from the group consisting of gram-positive and gram-negative bacteria. In some embodiments, the host cell is a gram-negative bacterium, such as *E. coli.* In certain embodiments, the host cell is *E. coli*, in particular *E. coli* BL21 (DE3) or other *E. coli* K12 or *E. coli* B834 derivatives. In further embodiments, the host cell is selected from the group consisting of *Escherichia coli* (*E. coli*), Pseudomonas, *Serratia marcescens*, Salmonella, Shigella (and other enterobacteriaceae), Neisseria, Hemophilus, Klebsiella, Proteus, Enterobacter, Helicobacter, Acinetobacter, Moraxella, Helicobacter, Stenotrophomonas, Bdellovibrio, Legionella, acetic acid bacteria, Bacillus, Bacilli, Corynebacterium, Clostridium, Listeria, Streptococcus, Staphylococcus, and Archaea cells. Suitable eukaryotic host cells are among others CHO cells, insect cells, fungi, yeast cells, e.g., *Saccharomyces cerevisiae*, *S. pombe*, *Pichia pastoris.*

In certain embodiments, the host cell is a prokaryotic cell, such as *E. coli,* in particular *E. coli* BL21 (DE3), *E. coli* BL21, *E. coli* K12, *E. coli* BLR, *E. coli* BL21 AI, *E. coli* BL21 pLysS, *E. coli* XL1 and *E. coli* DH5a. Further suitable *E. coli* strains include, but are not limited to DH1, DH5a, DM1, HB101, JmIOI-110, Rosetta(DE3)pLysS, SURE, TOP10, XLI-Blue, XL2-Blue and XLIO-Blue strains.

The transformed host cells are cultured under conditions suitable for expression of the nucleotide sequence encoding the fusion protein.

For producing the recombinant (poly)peptide of interest in form of the fusion proteins described herein, a vector of the invention can be introduced into a suitable prokaryotic or eukaryotic host organism by means of recombinant DNA technology (as already outlined above). For this purpose, the host cell is first transformed with a vector comprising a nucleic acid molecule according to the present invention using established standard methods (Sambrook, J. et al. (2001), supra). The host cell is then cultured under conditions, which allow expression of the heterologous DNA and thus the synthesis of the corresponding polypeptide. Subsequently, the (poly)peptide is recovered ("isolated") either from the cell or from the cultivation medium.

In various preferred embodiments of the invention, the fusion protein is not secreted. In such embodiments, it may preferably be expressed in form of inclusion bodies (lbs). In many cases, it may be useful to express the fusion protein in such an insoluble form, for example in cases where the peptide of interest is rather short, normally soluble and/or subject to proteolytic degradation within the host cell. Production of the peptide in insoluble form both facilitates simple recovery and additionally protects the peptide from the undesirable proteolytic degradation. In such embodiments, the third amino acid sequence may serve as a solubility tag, i.e., an inclusion body (IB) tag, that induces IB formation. Calcium ion (or earth alkaline metal ion) binding to the GG repeat(s) or any other change of environment may later catalyze the GG-repeat-facilitated folding of the fusion polypeptide into the native, active conformation, optionally with the calcium/earth alkaline metal ions acting as a folding helper/chaperone.

The terms "inclusion body" or "IB", as interchangeably used herein, relate to nuclear or cytoplasmic aggregates of substances, for instance proteins.

In various embodiments of the afore-mentioned methods, the fusion protein is recovered from the host cells in form of insoluble inclusion bodies, for example by any of the purification methods disclosed herein.

Renaturation may then be effected by exposing the isolated/purified fusion protein or the C-terminally modified (poly)peptide of interest (still including the third amino acid sequence fused to its N-terminus) to renaturation conditions. In some instances, this may include buffer conditions wherein the so-called refolding buffer comprises at least 0.01 mM, more preferably 0.01-40 mM of alkaline earth metal ions, in particular Ca²⁺. By virtue of the presence of GG repeats in the third amino acid sequence, such treatment triggers refolding of the polypeptide into its functional (native) conformation. Interestingly, the refolding of the third amino acid sequence also induces proper folding of the peptide/polypeptide of interest fused thereto. In various other embodiments, the renaturation/refolding thus occurs in the absence of calcium and/or earth alkaline metal ions in general. It may be preferred that the refolding occurs under reducing conditions, for example by using a reduction agent, such as TCEP ((tris(2-carboxyethyl)phosphine)).

The general advantages of expression of a fusion protein in form of inclusion bodies are set forth in greater detail in WO 2014/170430 A1, which is herewith included by reference in its entirety.

In various embodiments, the method also encompasses the purification of the fusion protein, wherein the fusion protein is purified using a method selected from affinity chromatography, ion exchange chromatography, reverse phase chromatography, size exclusion chromatography, and combinations thereof.

In several embodiments, the method may comprise the treatment of the fusion protein comprising a third amino acid sequence and a protease cleavage site between third amino acid sequence and (poly)peptide of interest with a protease suitable for cleavage of a protease cleavage site within the fusion protein. In preferred embodiments, the fusion protein is purified and, optionally, renatured prior to proteolytic cleavage using one or more methods disclosed above. Also after cleavage of the fusion protein, the method may comprise a further purification step as defined above.

In another aspect, the present invention also relates to an isolated (poly)peptide comprising a (poly)peptide of interest and the amino acid sequence C(X)ₙ fused to its C-terminus, wherein n is an integer of 1 to 100, and X is any amino acid with the exception of C, wherein said (poly)peptide of interest and said amino acid sequence C(X)ₙ are artificially combined. In various embodiments, the isolated (poly)peptide further comprises an amino acid sequence derived from an RTX protein, preferably derived from HlyA, that facilitates or increases renaturation of the (poly)peptide of interest fused to the N-terminus of the (poly)peptide of interest. All embodiments of the fusion protein disclosed above in relation to the inventive methods similarly apply to said isolated (poly)peptide and *vice versa.* This includes, in particular, the embodiments relating to the features of (poly)peptide of interest, the selection of (X)ₙ and the presence of a third amino acid sequence fused to the N-terminus of the (poly)peptide of interest.

All documents cited herein, are hereby incorporated by reference in their entirety. The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein and herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following examples and claims.

### Examples

### Methods

### NTCB-reaction

The lyophilized (poly)peptide was dissolved in 50 % acetonitrile (ACN) and the protein concentration was determined by Nanodrop. The solution was diluted to a concentration of 0.25 mg (poly)peptide/mL using 1 M ammonium acetate buffer (NH₄OAc) and the pH adjusted with aqueous NH₃. The obtained solution was diluted to a final concentration of ~800 mM ammonium acetate (NHaOAc) and a concentration of acetonitrile (ACN) of less than 10 %.
5 mM of tris(2-carboxyethyl)-phosphine hydrochloride (TCEP) were added to the sample and the sample was incubated for 15 minutes.

Then, 2-nitro-5-thiocyanatobenzoic acid (NTCB) was added in a 50-fold molar excess (stock solution: 125 mM in 6 M Guanidine-HCl (GuHCl)) relative to the (poly)peptide and incubated at different temperatures for different period of times.

The reaction was quenched with 1 mM cysteine in 6 M GuHCl (quenching solution) at different timepoints (30 seconds at room temperature, 1 hour at 37 °C, 2 hours at 37 °C, overnight at 37 °C).

### Target identification by RP-HPLC (Examples 1 and 3)

Column: Analytical CN,
Agilent Zorbax 300SB-CN 4.6 x 250 mm; 5 µm,
Elution: 5 % to 60 % ACN (acetonitrile) + 0.1% TFA (trifluoroacetic acid) in 20 minutes.
*Target identification by RP-HPLC (Example 2)*
Column: ACQUIRTY Premiere Peptide BEH C18, 300 Å, 1.7 µm, 2.1 × 100 mm,
Gradient: A: Water + 0.1 % FA (formic acid); B: ACN + 0.1 % FA (formic acid); 25 to 35 % B in 20.5 minutes,
Absorbance: 205 nm.

### Example 1

To determine whether the Cys-P1' position (the amino acid(s) C-terminal to the cysteine amino acid of the cysteine-containing tag) in the fusion protein influence NTCB-cleavage efficiency, a model peptide having the amino acid sequence FYGF or FGYF with different cysteine-containing tags was produced and the NTCB-reaction carried out as described above. The samples were subsequently subjected to RP-HPLC as described above.

Under the tested conditions, the starting peptide is fully activated (cyanylated) after 30 seconds and after 2 hours no more activated peptide is left in most samples (Table 1). Amidated peptide refers to the amidated peptide FYGF (SEQ ID NO:1) /FGYF (SEQ ID NO:2) where the Cys-containing tag is cleaved off, dehydroalanine refers to an undesired side product, activated peptide is the cyanylated but non-cleaved peptide and starting peptide refers to the non-cyanylated, non-cleaved peptide.

**Table 1: Overview of components in the sample.**

| **Overview** | | **% in sample (120 min reaction time)** | | | |
|---|---|---|---|---|---|
| **Target sequence** | **Tag** | **Amidated peptide** | **Dehydro-alanine** | **Activated peptide** | **Starting peptide** |
| FYGF | C | 13% | 0% | 79% | 0% |
| FYGF | CLW | 88% | 11% | 1% | 0% |
| FGYF | CK | 84% | 16% | 0% | 0% |
| FGYF | CE | 75% | 25% | 0% | 0% |
| FGYF | CL | 83% | 16% | 1% | 0% |
| FGYF | CW | 52% | 48% | 0% | 0% |
| FGYF | CGS | 56% | 44% | 0% | 0% |
| FGYF | CHHHHHH (SEQ ID NO:3) | 24% | 64% | 12% | 0% |

The results show that cysteine alone with further amino acids C-terminal thereto provides for very low yields and low cleavage efficiency. Highest yields were obtained for the tag sequences CLW, CL, CK and CE. Bulky amino acids, such as W appear to reduce the efficiency of the reaction. Surprisingly, the tag sequences CGS and CHis₆ also had comparably low yields.

Table 2 shows the differences in elution times for the product and the starting peptide as well as side products and intermediates. Higher differences allow better purification and are thus desirable.

**Table 2: Overview of elution differences (Δmin).**

| **Overview** | | **Elution difference (Δmin) relative to target** | | |
|---|---|---|---|---|
| **Target sequence** | **Tag** | **Starting peptide** | **Dehydro-alanine** | **Activated peptide** |
| FYGF | C | 1.3 | Not detected | 1 |
| FYGF | CLW | 5.71 | 5.48 | 5.74 |
| FGYF | CK | -0.36 | -0.26 | 0.43 |
| FGYF | CE | 0.97 | 1.08 | 1.63 |
| FGYF | CL | 3.66 | 3.99 | 4.19 |
| FGYF | CW | 4.59 | 4.9 | 4.9 |
| FGYF | CGS | 0.17 | 0.22 | -0.44 |
| FGYF | CHHHHHH | -1.65 | -0.65 | 1.47 |

### Example 2

To show that the method also works for longer target (poly)peptides, the FYGF/FGYF peptide target of Example 1 was replaced by a longer target sequence (45 amino acids, 4.26 kDa, pl 4.60). The tag used had the amino acid sequence CGGGGSGGGGS (SEQ ID NO:4). NTCB cleavage was carried out with CGGGGSGGGGS-Tag at pH 8, 9 and 10 at adjusted conditions (0.5mg/mL solution in 6M GuHCl, TCEP reduction followed by addition of solid 10x excess NTCB and addition of NH₃ (25% aq) to 1M final concentration). After the reaction no starting material was found and target containing C-terminal amidation was found as a major species aside from dehydroalanine and other side products.

### Example 3

A fusion protein of an N-terminal RTX-derived sequence, a (poly)peptide of interest (31 amino acids, 3.57 kDa, pl 9.50) and the C-terminal tag sequence CGS was prepared. The fusion protein was expressed in E.coli in form of inclusion bodies and after isolation solubilized in 8M urea. After centrifugation to remove insoluble parts, NTCB cleavage at pH 9 was carried out under adapted conditions. After TCEP reduction (5mM; 60min) solid NTCB was added (50x molar excess) followed by aqueous ammonia (final concentration: 1M) and pH adjustment to pH 9 by addition of acetic acid. After incubation at 37°C for 60min a desalting column equilibrated with 8M urea was used to remove excess NTCB and its side products. The obtained protein solution was renaturated. After renaturation, the fusion protein with the amidated C-terminus was cleaved with TEV protease to remove the N-terminal RTX-derived sequence. The proteolytic cleavage solution showed several peaks of released pepteins separated from proteinogenic contents. Among all pepteins the main species (45% acc. to HPLC peak area) belonged to the C-terminal amidated target. Chromatographic purification yielded the pure amidated peptide.

## Claims

1. Method for the C-terminal modification of a (poly)peptide of interest, wherein said (poly)peptide of interest is free of cysteine residues and at least 10 amino acids in length, said method comprising:
a) providing a fusion protein comprising the (poly)peptide of interest with the amino acid sequence C(X)ₙ fused to its C-terminus, wherein n is an integer of 1 to 100, and X is any amino acid with the exception of C, wherein the fusion protein is an artificial, non-natural protein or fragment thereof in that the C-terminal amino acid sequence C(X)ₙ does not naturally occur in combination with the (poly)peptide of interest;
b) reacting the fusion protein with 2-nitro-5-thiocyanatobenzoic acid (NTCB) or salt thereof to cyanylate the cysteine residue;
c) contacting the cyanylated fusion protein with a nucleophile selected from the group consisting of ammonia, an ammonium salt or a primary amino group containing agent to cleave the cyanylated fusion protein and obtain the C-terminally modified (poly)peptide of interest.

2. The method of claim 1, wherein
(1) steps b) and c) are carried out at a pH of 8.0-10.0, preferably at pH 9.0; and/or
(2) steps b) and c) are carried out simultaneously, preferably by adding NTCB and the nucleophile at the same time.

3. The method of claim 1 or 2, wherein in step c) ammonia or ammonium hydroxide is used as the nucleophile and the C-terminal modification is an amidation.

4. The method of any one of claims 1 to 3, wherein
(1) n is 1 to 50, preferably 1 to 20 or 1 to 15 or 1 to 10 or 1 to 5 or 1 to 2 or n is 1; and/or
(2) n is selected such that the amino acid sequence C(X)ₙ has a length of at least 5% of the total length of the (poly)peptide of interest.

5. The method of claim 1 or 2, wherein in step c) a monomeric amino acid or a peptide of 2 to 100 amino acids, preferably 2 to 50 or 2 to 25 or 2 to 15 amino acids, in length is used as the amino group containing agent and the C-terminal modification is a C-terminal elongation by said amino acid or peptide, wherein said amino acid is not C, and preferably not G, or said peptide does not contain a C residue.

6. The method of claim 5, wherein n is 1 or n is selected such that the C-terminal tag consisting of the amino acid sequence C(X)ₙ differ in length by at least 1, preferably by at least 2 amino acids.

7. The method of claim 6, wherein said difference in length is at least 5% of the total length of the (poly)peptide of interest.

8. The method of any one of claims 1 to 7, wherein the (poly)peptide of interest is 10 to 1000 amino acids in length, preferably 10 to 500 amino acids or 15 to 250 amino acids or 20 to 150 amino acids or 20 to 100 amino acids or 20 to 50 amino acids.

9. The method of any one of claims 1 to 8, wherein the method further comprises the step of purifying or enriching the C-terminally modified (poly)peptide of interest, preferably relative to the non-modified (poly)peptide of interest, wherein purification or enrichment is optionally carried out by chromatography, preferably size exclusion chromatography, affinity chromatography or HPLC.

10. The method of any one of claims 1 to 9, wherein the fusion protein and the C-terminally modified (poly)peptide of interest obtained in step c) differ in length by at least 1 amino acid, preferably by at least 2 amino acids, preferably the fusion protein differs from the C-terminally modified (poly)peptide of interest in molecular mass by at least 2.5%, preferably by at least 5%.

11. The method of any one of claims 1 to 10, wherein the fusion protein is recombinantly produced in a host organism.

12. The method of any one of claims 1 to 11, wherein the fusion protein further comprises an amino acid sequence derived from an RTX protein, preferably derived from HlyA, that preferably facilitates renaturation of the (poly)peptide of interest fused to the N-terminus of the (poly)peptide of interest.

13. The method of any one of claims 1 to 12, wherein the amino acid sequence (X)ₙ is X¹(X)ₙ₋₁, wherein
(1) X¹ is not W, preferably X¹ is not W, Y, F, or P; and/or
(2) X¹ is an unpolar amino acid, preferably A, V, L or I, more preferably L, a basic amino acid, preferably K or R, more preferably K, or an acidic amino acid, preferably D or E.

14. The method of any one of claims 1 to 13, wherein the (poly)peptide of interest has biological activity, preferably in a mammal, more preferably in a human, or is useful as a pharmaceutical for a mammal, preferably a human.

15. Isolated (poly)peptide comprising a (poly)peptide of interest and the amino acid sequence C(X)ₙ fused to its C-terminus, wherein n is an integer of 1 to 100, and X is any amino acid with the exception of C, wherein said (poly)peptide is an artificial (poly)peptide in that the (poly)peptide of interest and said amino acid sequence C(X)ₙ are artificially combined, and wherein the isolated (poly)peptide optionally further comprises an amino acid sequence derived from an RTX protein, preferably derived from HlyA, that facilitates or increases renaturation of the (poly)peptide of interest fused to the N-terminus of the (poly)peptide of interest.
